# EUROPEAN PATENT APPLICATION

(11) **EP 3 092 957 A1**
(43) Date of publication of application: **16.11.2016**
(21) Application number: 16168626.6
(22) Date of filing: 06.05.2016
(51) Int. Cl.: A61B 17/12, A61B 90/00, A61F 2/01

(54) **ELECTROLYTIC DETACHMENT WITH FLUSH SYSTEM FOR IMPLANT DELIVERY**

(30) Priority: 11.05.2015 US 201514708688
(71) Applicant: Covidien LP, Mansfield, MA 02048 (US)
(72) Inventor: Devino, Vincent, Mission Viejo, CA 92692 (US); Kadam, Madhur, Lake Forest, CA 92630 (US)
(74) Representative: Gray, James

(57) **Abstract**

Detachment of a medical device from a delivery assembly can be electrolytic and provide flushing with an infusion of fluid to enhance detachment procedures. A device providing such capabilities can include a catheter having a lumen; a core member extending through the lumen; an implant attached to the core member by an electrolytic detachment junction (30), the detachment junction being radially adjacent to an electrode (120) within the lumen; and a pump in fluid communication with a distal end region of the catheter through the lumen, the pump being configured to provide a flow of a fluid between the return electrode and the detachment junction. The implant can be positioned at a target location. The electrode can be positioned radially adjacent to the detachment junction. A voltage potential is applied while a fluid is flushed between the detachment junction and the electrode through the lumen of the catheter.

## Description

### Field

The subject technology relates to delivery of implantable devices by a delivery system.

### Background

The use of endovascular techniques for the implantation of medical devices and the occlusion of body cavities such as arteries, veins, fallopian tubes, or vascular deformities is known in the art. For example, occlusion of vascular aneurysms can be performed using an implantable device, such as an intrasaccular implant, that is introduced with the aid of an endovascular delivery wire through a catheter. Once moved to the treatment site, the intrasaccular implant can be moved into the aneurysm cavity to occlude the aneurysm.

The severance of the implant from the delivery wire can be problematic. On the one hand, the device must be capable of forming as small profile as possible to be guided through the fine bore of the catheter to its destination, while on the other hand it must bring about a reliable severance of the implant. Absent a reliable severance of the implant, withdrawal of the delivery wire and catheter may cause unintended removal of the implant from the cavity to be occluded and thus injure and/or rupture of the wall of the cavity or vessel.

Traditional mechanical methods for the severance of implants from the insertion means are reliable. However, the necessary rigidity of the connection between the implant and the delivery means can impede the introduction of the implant. Furthermore, the low load carrying capacity of the connection due to its rigidity entails an appreciable risk of premature detachment of the insertion means from the occluding implant. Moreover, in the case of mechanical separation of the delivery wire and the implant, mechanical energy must be transmitted (e.g., by rotation of the delivery wire), which may cause the implant to be dislodged out of the correct position.

Traditional electrolytic severance of the implant involves using an electrolytically corrodible design on the end of the delivery wire at the connection between the delivery wire and the implant. Such a device elegantly makes use of the voltage applied to the implant serving as an anode for electrothrombosis. However, the connection of the implant to the delivery wire is limited by the requirements of the electrolytically corrodible region. For example, the only materials that can be utilized are those which have a sufficiently high degree of strength to enable reliable guidance of the occluding wire through the delivery wire. The selection of materials for forming the point of eventual electrolytic severance is consequently extremely limited.

In the case of traditional devices for the electrolytic severance of implants, the implant and the delivery wire are not produced integrally, but instead are produced mechanically connected to each other. This design has the inherent disadvantage that the delivery wire must be tapered toward its end in an involved grinding operation in order to ensure sufficient strength in the proximal zone of the delivery wire while facilitating electrolytic, corrosive severance of the wire end at the distal part of the delivery wire connected to the implant. In order to ensure sufficient strength of the connection point, the corrodible zone of the end of the delivery wire must not have a diameter below a certain minimum value since it is subjected to a high flexural load. The corrodible wire end representing the connection point between the intrasaccular implant and the delivery wire can be consequently extremely rigid and require a relatively long time for electrolytic corrosive severance.

### Summary

Electrolytic severance of an implantable medical device can involve using an electrolytically corrodible design on the end of a delivery wire at the connection between the delivery wire and the medical device.

According to some embodiments of the subject technology, a system for delivering an implant, includes a catheter having a lumen; a core member extending through the lumen; an implant attached to the core member by an electrolytic detachment junction, the detachment junction being radially adjacent to an electrode within the lumen; and a pump in fluid communication with a distal end region of the catheter through the lumen, the pump being configured to provide a flow of a fluid between the return electrode and the detachment junction.

The electrode can be attached to an inner surface of the catheter. The electrode can form a ring annularly extending along an inner surface of the catheter. The core member can be disposed along a central axis of the catheter. A stabilization member can extend radially into the lumen from an inner surface of the catheter and contacting the core member. The return electrode and the detachment junction can be electrically connected to a power source. The detachment junction can be electrolytically corrodible. The detachment junction can be of a material that is more susceptible to electrolytic corrosion than a material of the core member or a material of the implant. The return electrode can be disposed on a distal end of a conductive return path member extending along the catheter. At least a portion of the core member can be electrically insulated on an outer surface thereof. A proximal portion of the core member, proximal to the detachment junction, can have a cross-sectional dimension greater than a cross-sectional dimension of the detachment junction.

According to some embodiments of the subject technology, a method of detaching an implant, includes positioning the implant, the implant being attached to a core member having a detachment junction; positioning a catheter such that a return electrode of the catheter can be radially adjacent to the detachment junction; and applying a voltage potential across the electrolytic detachment junction and the return electrode and, while applying the voltage potential, flushing a fluid between the detachment junction and the return electrode through the lumen of the catheter.

Applying the voltage potential can include applying a first charge to the detachment junction via the core member, and applying a second charge, opposite the first charge, to the return electrode. Positioning the return electrode can include positioning the return electrode radially about the detachment junction. Positioning the implant can include advancing the implant through a lumen of the catheter. Positioning the return electrode can include advancing the catheter along the core member. Applying the voltage potential can include applying the voltage potential until the detachment junction has corroded. The voltage can be applied until the implant is separated from the core member. The fluid can be flushed until the implant is detached from the core member.

Additional features and advantages of the subject technology will be set forth in the description below, and in part will be apparent from the description, or may be learned by practice of the subject technology. The advantages of the subject technology will be realized and attained by the structure particularly pointed out in the written description and claims hereof as well as the appended drawings.

It is to be understood that both the foregoing general description and the following detailed description are exemplary and explanatory and are intended to provide further explanation of the subject technology as claimed.

### Brief Description of the Drawings

The accompanying drawings, which are included to provide further understanding of the subject technology and are incorporated in and constitute a part of this description, illustrate aspects of the subject technology and, together with the specification, serve to explain principles of the subject technology.
FIG. 1A shows a perspective view providing an overview of a delivery system, in accordance with one or more embodiments of the present disclosure.
FIG. 1B shows a view providing an overview of a delivery system with respect to a patient, in accordance with one or more embodiments of the present disclosure.
FIG. 1C shows a perspective view providing an overview of a delivery system, in accordance with one or more embodiments of the present disclosure.
FIG. 2 shows a perspective side view of a braid ball implant, in accordance with one or more embodiments of the present disclosure.
FIG. 3 shows a side-sectional view of a braid ball implant deployed within a bifurcation aneurysm, in accordance with one or more embodiments of the present disclosure.
FIG. 4 shows a side view of a distal end of a delivery system, in accordance with one or more embodiments of the present disclosure.
FIG. 5 shows a sectional view of a distal end of a delivery system, in accordance with one or more embodiments of the present disclosure.
FIG. 6A shows a side view of a delivery catheter, in accordance with one or more embodiments of the present disclosure.
FIG. 6B shows a sectional view of a delivery catheter, in accordance with one or more embodiments of the present disclosure.
FIG. 6C shows a sectional view of a delivery catheter, in accordance with one or more embodiments of the present disclosure.
FIG. 6D shows a sectional view of a delivery catheter, in accordance with one or more embodiments of the present disclosure.
FIG. 7 shows a side-sectional view of a stage of implant deployment within a bifurcation aneurysm, in accordance with one or more embodiments of the present disclosure.
FIG. 8 shows a side-sectional view of a stage of implant deployment within a bifurcation aneurysm, in accordance with one or more embodiments of the present disclosure.
FIG. 9 shows a side-sectional view of a stage of implant deployment within a bifurcation aneurysm, in accordance with one or more embodiments of the present disclosure.
FIG. 10 shows a side-sectional view of a stage of implant deployment within a bifurcation aneurysm, in accordance with one or more embodiments of the present disclosure.

### Detailed Description

In the following detailed description, specific details are set forth to provide an understanding of the subject technology. It will be apparent, however, to one ordinarily skilled in the art that the subject technology may be practiced without some of these specific details. In other instances, well-known structures and techniques have not been shown in detail so as not to obscure the subject technology.

In accordance with some embodiments disclosed herein is the realization that detachment of a medical device from a delivery assembly can be improved by enhancing features to focus the electrolytic corrosion activity. Thus, various embodiments provide for detachment zones that can facilitate electrolytic detachment of a delivery mechanism, making the detachment process faster and more reliable.

The medical device can be implanted in body cavities or blood vessels. In addition to the medical device, the delivery system can comprise a voltage source, a cathode, and a catheter. The medical device can be slid in the catheter in the longitudinal direction. A delivery wire may engage the medical device and be adapted to serve as an anode, such that a portion of the delivery wire is designed to be electrolytically corroded at one or more points so that while in contact with a body fluid, one or more portions of the medical device may be released from the delivery wire.

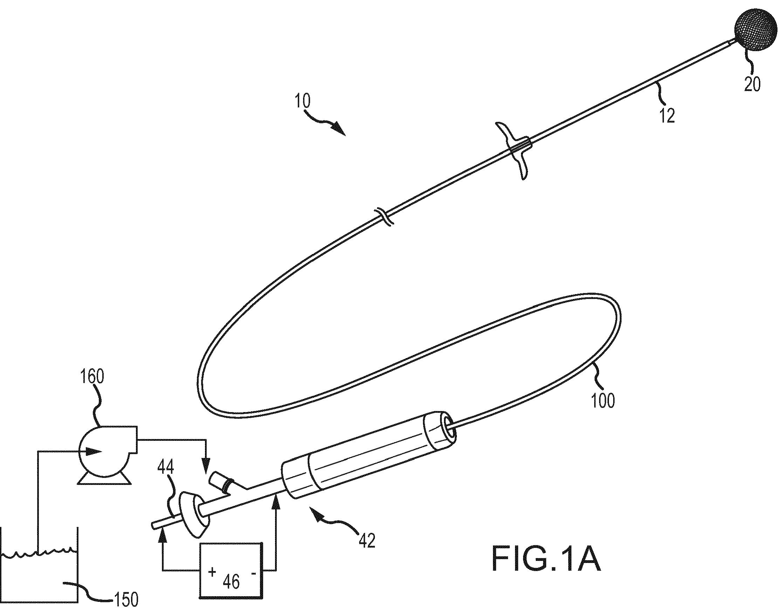

According to some embodiments, FIG. 1A presents an overview of a delivery system 10 including an implant 20 and a handle 42. The handle 42 shown provides proximal access to a delivery wire 44 that engages the implant 20 at a distal end thereof. The catheter/pusher shaft 12 can include a simple extrusion (e.g., PTFE, FEP, PEEK, etc.) or can be constructed using conventional catheter construction techniques and include a liner, braid support and outer jacket (not shown). A loading sheath or delivery catheter 100 is typically provided over the shaft of a pusher 12.

A power supply 46 may be coupled to a proximal portion of the delivery wire 44. The power supply 46 may also be coupled to a proximal portion of the handle 42 or to the patient. A current can flow from the power supply 46, to a detachment zone at or near the implant 20, and to a return path via the catheter shaft 100 (and/or another structure extending near the detachment zone. Power supply 46 may be a direct current power supply, an alternating current power supply, or a power supply switchable between a direct current and an alternating current. A positive terminal of a direct current power supply, as shown in FIG. 1A, may be coupled to the proximal portion of the delivery wire 44 and a negative terminal of a direct current power supply may be coupled to the proximal portion of the handle 42.

Power supply 46 may provide a current through the delivery system 10 to initiate an electrolytic process during use of the assembly in a fluid medium such as a bloodstream, which may be used as an electrolyte, as discussed further herein. A power supply, such as an alternating or direct current power supply, may additionally be used to initiate an electrothrombosis process. According to some embodiments, the power supply 46 can include an electrical generator configured to output medically useful electrical current. The power supply 46 can include a suitable controller that can be used to control various parameters of the energy output by the generator, such as intensity, amplitude, duration, frequency, duty cycle, polarity, etc. For example, the power supply 46 can provide a voltage of about 12 volts to about 28 volts and a current of about 1 mA to about 2 mA.

According to some embodiments, as shown in FIG. 1A, a fluid source 150 may be provided in connection with a pump 160 for infusion of the fluid via the delivery catheter 100. The fluid of the fluid source 150 can be saline or another sterile solution suitable for infusion into a patient. The fluid of the fluid source 150 can be an electrolyte solution. The fluid can include saline. The fluid can be infused with a drug (e.g., Heparin). The fluid can facilitate electric conduction therein. The fluid may be drawn from the fluid source 150 into the pump 160 and provided to a lumen of the delivery catheter 100. The pump 160 can be an infusion pump, a pressurized container, and/or a gravity-based infusion mechanism. Appropriate pathways and interfaces may be provided between the fluid source 150, the pump 160, and the catheter 100.

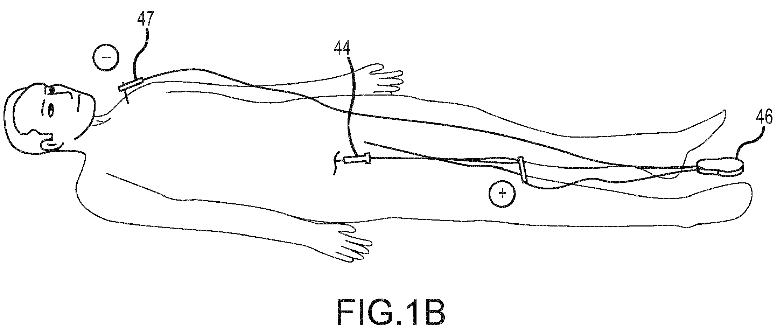

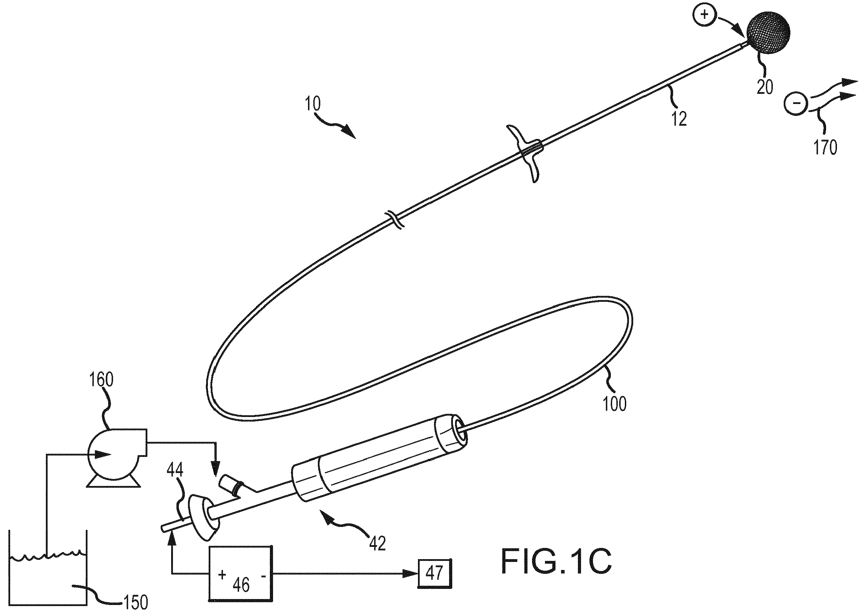

According to some embodiments, as shown in FIGS. 1B and 1C, the current from the detachment zone may flow to the patient, and subsequently to ground or to the power supply 46. For example, a terminal of the power supply 46 can be connected to the patient at a region 47 on the surface of the patient's skin to provide a conductive pathway from the detachment zone at or near the implant 20 to ground or to the power supply 46. A first charge at the detachment zone at or near the implant 20 can occur where an opposite charge is induced in the fluid flow 170 at or near the detachment zone.

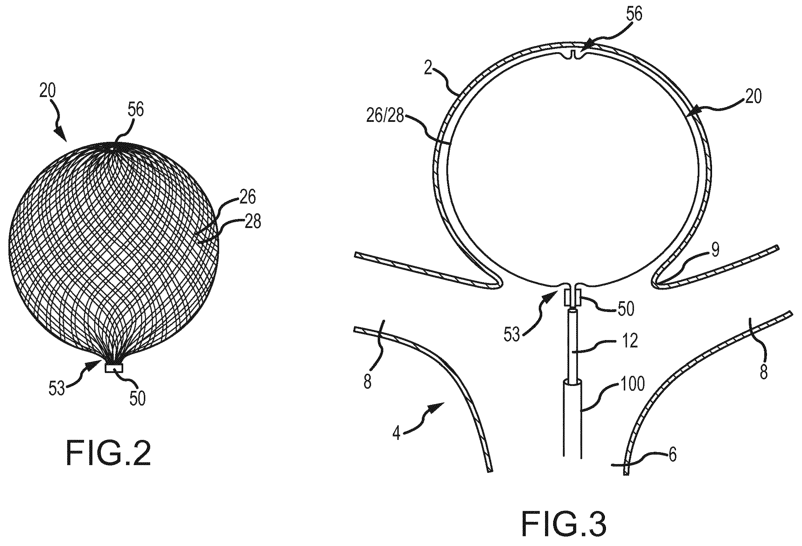

According to some embodiments, as shown in FIGS. 2 and 3, an implant 20 delivered by the system 10 can be a braid ball implant. The braid ball implant 20 can be formed from tubular braid stock including a resilient material, such as Nitinol, that defines an open volume (generally round, spherical, ovular, heart-shaped, etc.) in an uncompressed/unconstrained state. The size of the implant can be selected to fill an aneurysm 2, so the proximal end 53 of the braid ball implant 20 helps direct blood flow along the surface of the braid from which it is constructed to the branch vessels 8. A distal end 56 of the ball can be dome-shaped. The braid ball implant 20 can include a single layer or two layers 26, 28 (inner and outer layer, respectively) construction at least where impacted by flow at the neck 9 of the aneurysm 2. As shown, one or more turns of a coil (e.g., Pt wire) or a band (not shown) can provide a distal radiopaque feature to mark the location of the implant 20. Some exemplary implants that can be used in conjunction with the systems described herein are disclosed at U.S. Pub. No. 2013/0123830, published on May 16, 2013, the entirety of which is incorporated herein by reference.

According to some embodiments, the implant 20 can include a hub 50 at a proximal end 53 thereof. The hub 50 can be fixedly attached to the remainder of the implant 20. For example, the hub 50 can grasp braided filaments of the layers 26, 28 of the implant 20.

According to some embodiments, the implant 20 can be set within an aneurysm sac 2 at a vascular bifurcation 4, formed by trunk vessel 6 and efferent vessels 8. The implant 20 can be delivered by access through the trunk vessel 6 (e.g., the basilar artery), preferably through a commercially available microcatheter with a delivery system as detailed below. To deliver the implant 20, the pusher sleeve 12 is positioned such that the implant 20 can be delivered at least partially into the aneurysm sac 2. Finally, the pusher sleeve 12 is withdrawn into the delivery catheter 100.

While the implant 20 can be a braid ball implant as illustrated herein, the implant 20 can have any other form or structure, according to various embodiments. For example, the implant 20 can be a vasoocclusive coil, a cylindrical, tube-like stent, or a filter. Other types of implants and treatment devices are generally known. The subject technology can be applied to any such implant or treatment device for delivery and detachment thereof. For example, a given implant can include a hub 50 for engagement and release by a delivery system, as disclosed further herein.

Traditional electrolytic detachment members are generally a single wire with a constant diameter. Detach wires can be drawn and provide high corrosion resistance due to a crystalline structure. Generally, when such detach wires are used they leave behind small particulate and these particulate can interfere with MRI imaging and also could lead to secondary stroke if particulate flows to distal vessel. Detachment time can be reduced by concentrating erosion to a limited area.

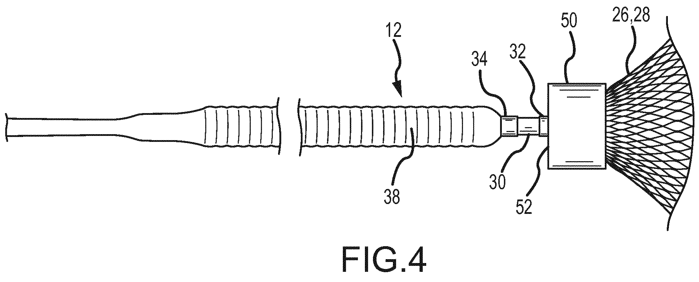

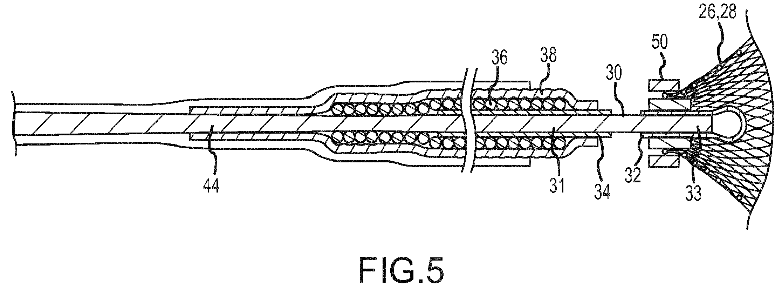

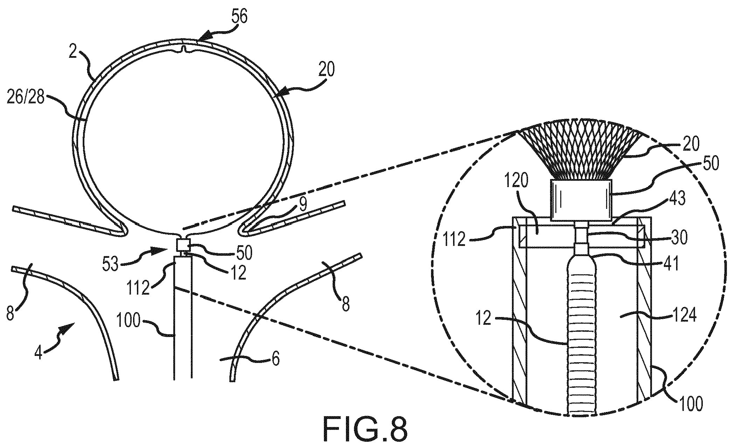

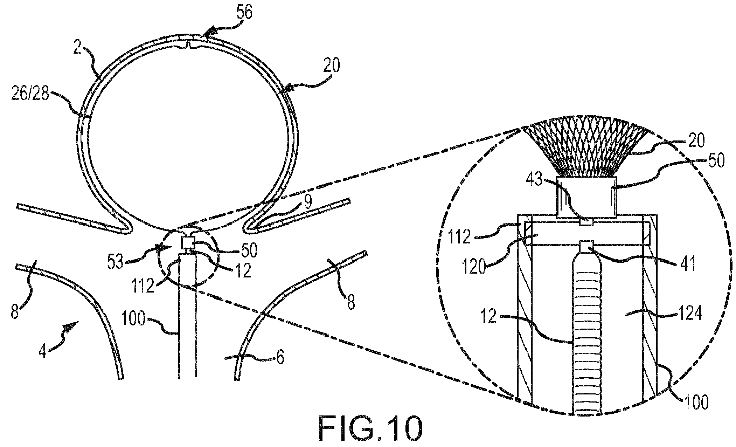

According to some embodiments, as shown in FIG. 4 and 5, a delivery system 10 includes a delivery wire 31 (e.g., core member, etc.), an implant wire 33, and a detachment zone 30 between the delivery wire 31 and the implant wire 33. The detachment zone 30 can represent the joining of a distal end 40 of the delivery wire 31 and a proximal end 43 of the implant wire 33 (see FIGS. 8 and 10). The types and methods of joining the delivery wire 31 and the implant wire 33 across the detachment zone 30 are discussed further herein.

According to some embodiments, portions of the delivery wire 31 can be coated with a nonconductive material. A proximal insulating layer 34 can be provided over at least a portion of an outer surface of the delivery wire 31. For example, the proximal insulating layer 34 can circumferentially surround an outer surface of the delivery wire 31. According to some embodiments, a distal insulating layer 32 can be provided over at least a portion of an outer surface of the implant wire 33. For example, the distal insulating layer 32 can circumferentially surround and contact an outer surface of the implant wire 33.

According to some embodiments, proximal and distal insulating layers 34, 32 leave exposed the detachment zone 30 between the delivery wire 31 and the implant wire 33. When in contact with a body fluid, such as blood, the fluid serves as an electrolyte allowing current to be focused on the non-coated detachment zone 30. The proximal and distal insulating layers 34, 32 prevent exposure of the delivery wire 31 and the implant wire 33 to the fluid. Accordingly, electrical energy conducted along the pusher wire 44 is concentrated at the detachment zone 30, thereby reducing the time required to erode away the detachment zone 30. The proximal and distal insulating layers 34, 32 can be over-molded, co-extruded, sprayed on, or dip-coated with respect to the delivery wire 31 and/or the implant wire 33.

The proximal and distal insulating layers 34, 32 can be of an electrically nonconductive or insulative polymer, such as polyimide, polypropylene, polyolefins, combinations thereof, and the like. Laser ablation can be employed to selectively remove the coating to a controlled length minimizing the time required to erode through the component. Lengths as small as 0.0005" and as large as 0.1" or longer can be removed. According to some embodiments, lengths of detachment zone 30 can be greater than 0.005" and/or less than 0.010" to provide sufficient exposure to achieve detachment times of less than 30 seconds.

At least a portion of the delivery wire 31, the implant wire 33, and/or the detachment zone 30 can be coated with a conductive material, such as carbon, gold, platinum, tantalum, combinations thereof, and the like. One or more metallic coatings can be applied using known plating techniques.

The delivery wire 31, the implant wire 33, and/or components of the detachment zone 30, can include one or more of the following materials: ceramic materials, plastics, base metals or alloys thereof, and preferably stainless steel. Some of the most suitable material combinations for forming the electrolytically corrodible points can include one or more of the following: stainless steels, preferably of the type AISI 301, 304, 316, or subgroups thereof; Ti or TiNi alloys; Co-based alloys; noble metals; or noble metal alloys, such as Pt, Pt metals, Pt alloys, Au alloys, or Sn alloys. Further, ceramic materials and plastics employed for forming the medical device can be electrically conductive.

Other features and discussion of electrolytically corrodible connections is provided in other applications of the present assignee, including the discussion and disclosure of U.S. Patent Application Publication No. 2012/0010648 and U.S. Patent Nos. 7,323,000, and 8,048,104, the entirety of each of which is incorporated herein by reference.

Electrolytically non-corrodible sections of the delivery wire can contain one or more of the following materials: noble metals or noble metal alloys, corrosion-resistant ceramic materials, corrosion-resistant plastics, and/or platinum metal alloys. The use of the above mentioned materials for the formation of electrolytically non-corrodible sections and of the electrolytically corrodible flanges ensures specific electrolytic corrosion of the flanges at the predetermined points.

In accordance with some embodiments, the electrolytically corrodible detachment zone can also be pre-corroded by etching or other methods. Thus, the structure(s) of a given cross-sectional profile can be modified to reduce the presence of corners, increase the recess depth, and/or otherwise enhance the corrosion rate. Further, various excellent structural designs can be provided to achieve desired corrosion performance through the teachings disclosed herein without pre-corrosion of the corrodible points.

Some embodiments can include a corrodible detachment zone that has a partial coating of a material to provide a greater or lesser electrochemical resistance. Thus, in embodiments that have one or more corrodible points, the electrochemical resistance of the points can be varied to achieve staged or preferential electrochemical resistance. Coatings of Zn, Sn, or alloys of such metals on fittings of stainless steel have been found to be particularly satisfactory.

As shown in FIG. 5, the distal insulating layer 32 electrically isolates the implant 20 from an electrical charge conducted along a length of the delivery wire 31 and the implant wire 33. A proximal end of the distal insulating layer 32 may be positioned at or proximal to the hub 50, and a distal end of the distal insulating layer 32 may be positioned at or distal to the hub 50. Likewise, a proximal end of the implant wire 33 may be positioned proximal to the hub 50, and a distal end of the implant wire 33 may be positioned within or distal to the hub 50.

According to some embodiments, a marker coil 36 is wound helically about an outer surface of the proximal insulating layer 34. The marker coil 36 can be of a radiopaque material, such as platinum, gold, palladium, iridium, and alloys thereof. An insulative layer 38 can be provided about an outer surface of the marker coil 36. For example, as shown in FIG. 5, the insulative layer 38 can extend over an entire length of the marker coil 36 and distally beyond the marker coil 36, such that every portion of the marker coil 36 is covered by the insulative layer 38. A distal end of the insulative layer 38 may contact and/or be adhered to the proximal insulating layer 34. The insulative layer 38 can be of an insulative biocompatible polymer material, such as polytetrafluoroethylene (PTFE). The insulative layer 38 may be shrink-wrapped over the corresponding portion of the delivery wire.

According to some embodiments, as shown in FIG. 5, a pusher wire 44 can be integrally connected to the delivery wire 31. Accordingly, an electric charge applied to the pusher wire 44 can be conducted through the pusher wire 44, the delivery wire 31, and the detachment zone 30. Furthermore, an axial force applied to the pusher wire 44 can result in an axial movement of the delivery wire 31 and the implant 20.

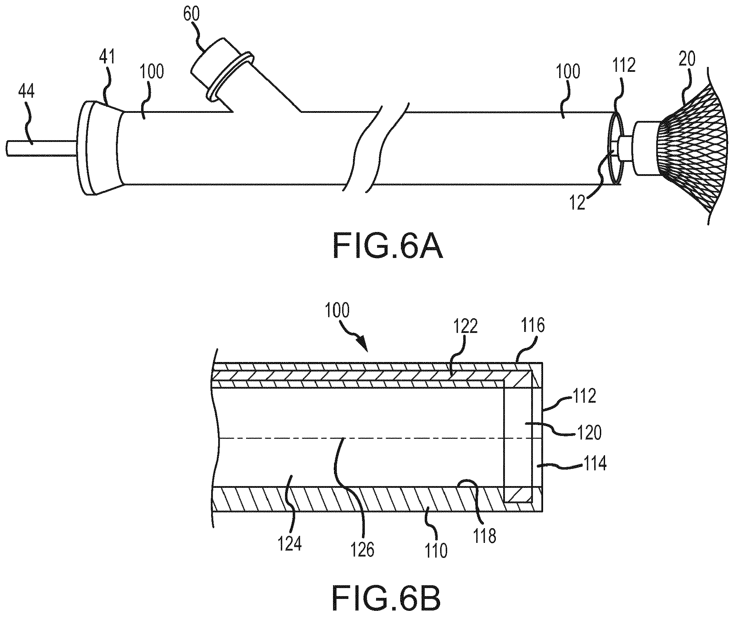

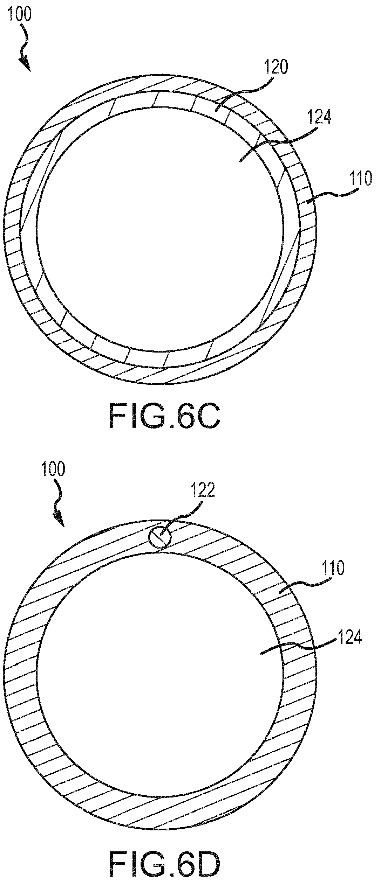

Referring now to FIGS. 6A-D, with continued reference to FIGS. 1A-5, illustrated are various views of an exemplary delivery catheter, according to one or more embodiments of the subject technology. More particularly, FIG. 6A depicts a side view of a delivery catheter 100, FIG. 6B depicts a sectional view of the delivery catheter 100, FIG. 6C depicts a sectional view of the delivery catheter 100, and FIG. 6D depicts a sectional view of the delivery catheter 100. The delivery catheter 100 may be similar in some respects to the delivery catheter 100 of FIGS. 1A and 3 and therefore may be best understood with reference thereto, where like numerals indicate like elements or components not described again in detail. Similar to the delivery catheter 100 of FIGS. 1A and 3, for example, the delivery catheter 100 may contain the pusher wire 12 and/or the implant 20.

According to some embodiments, as shown in FIG. 6A-B, the delivery catheter 100 can be formed as a generally tubular member with a body 110 extending along and about a central axis 126 and terminating in a distal end 112. According to some embodiments, delivery catheter 100 is generally constructed to track over a conventional guidewire beyond the handle 42 in the cervical anatomy and into the cerebral vessels associated with the brain and may also be chosen according to several standard, "microcatheter" designs that are generally available. Accordingly, delivery catheter 100 has a length that is at least 125 cm long, and more particularly may be between about 125 cm and about 175 cm long. Typically the delivery catheter 100 is about 155 cm long. Inner lumen 36 of the delivery catheter generally has an inner diameter between about 0.01 inch and about 0.098 inch (0.25-2.49 mm). Other designs and dimensions are contemplated. Commercially available microcatheters which may be suitable for use as delivery catheters include the REBAR™ Reinforced Micro Catheter, which is available from Covidien LP and the MARKSMAN™ Catheter, which is available from Covidien LP.

According to some embodiments, the body 110 of the delivery catheter 100 can be made from various thermoplastics, e.g., polytetrafluoroethylene (PTFE or TEFLON®), fluorinated ethylene propylene (FEP), high-density polyethylene (HDPE), polyether ether ketone (PEEK), etc., which can optionally be lined on the inner surface of the catheter or an adjacent surface with a hydrophilic material such as polyvinylpyrrolidone (PVP) or some other plastic coating. Additionally, either surface can be coated with various combinations of different materials, depending upon the desired results.

According to some embodiments, the delivery catheter 100 can have a proximal end 41, a distal end 112, and an inner lumen 124 extending from a proximal port 44 of the delivery catheter 100. The proximal port 44 of the delivery catheter 100 may be provided with an adapter (not shown) having a hemostatic valve. The delivery catheter 100 is generally constructed to bridge between a femoral artery access site and a cervical region of the carotid or vertebral artery and may be chosen according to several standard designs that are generally available. Accordingly, the delivery catheter 100 may be at least 85 cm long, and more particularly may be between about 95 cm and about 105 cm long. Further to conventional and available designs, inner lumen 43 of guide catheter 13 generally has an inner diameter that is between about 0.038 inch and 0.090 inch (0.88-2.29 mm), and more particularly may be between about 0.052 inch and about 0.065 inch (1.32-1.65 mm). Other designs and dimensions are contemplated.

According to some embodiments, an infusion fluid can be provided to an infusion port 60, shown in FIG. 6A, to provide fluid communication to the distal end region of the delivery catheter 100. Infusion may be accomplished by a pump, a syringe, or other fluid control mechanism. The infusion port 60 can be provided with fluid communication to a distal end region of the lumen 124 of the delivery catheter 100.

According to some embodiments, an electrode 120 is provided at a distal end region of the delivery catheter 100. According to some embodiments, as shown in FIG. 6B, the electrode 120 can form an annular ring that extends entirely circumferentially about the central axis 126. Alternatively or in combination, the electrode 120 can extend less than entirely circumferentially. For example, the electrode 120 may be entirely disposed on one radial side of the central axis 126. By further example, the electrode 120 may provide a plurality of discrete, noncontiguous sections about the central axis 126. Such sections of the electrode 120 can be in electrical communication with each other or separately powered. According to some embodiments, as shown in FIG. 6B, the electrode 120 can be displaced from the distal end 112 of the delivery catheter 100 by a distal section 114 of the body 110. Alternatively or in combination, a distal portion of the electrode 120 can extend to the distal end 112 of the delivery catheter 100, such that the electrode 120 forms a portion of the distal end 112. According to some embodiments, as shown in FIG. 6B, an inner surface of the electrode 120 can be flush with an inner surface 118 of the body 110. Alternatively or in combination, the inner surface of the electrode 120 can extend more radially inwardly relative to the inner surface 118 of the body 110(e.g., providing a "step"). Alternatively or in combination, the inner surface of the electrode 120 can extend less radially inwardly relative to the inner surface 118 of the body 110 (e.g., be recessed into the body). According to some embodiments, as shown in FIG. 6B, the electrode 120 can be surrounded radially by an outer section 116 of the body 110 to provide insulation from an external environment. The electrode 120 can be a band, as shown in FIG. 6B. The electrode 120 can be a wire or coil embedded in the wall of the body 110

The electrode 120 can include one or more rings, one or more coils or other suitable conductive structures, and can each form an inner surface that is exposed and configured for electrical activity. The electrode 120 can have a fixed inner diameter or size, or a radially expandable inner diameter or size. The electrode 120 can be a "painted" electrode. The electrode can include platinum, platinum alloys (e.g., 92% platinum and 8% tungsten, 90% platinum and 10% iridium), gold, cobalt-chrome, stainless steel (e.g., 304 or 316), and combinations thereof.

According to some embodiments, as shown in FIG. 6B and 6D, the electrode 120 can be electrically connected to the power source 46 via a conductive connection line 122. The connection line 122 can extend proximally along or within the body 110 to the proximal end 41 of the delivery catheter 100. The connection line 122 can include more than one line extending within the body 110. According to some embodiments, the connection line 122 can form a helical coil along or within at least a portion of the body 110. Alternatively or in combination, the connection line 122 can form a braided, woven, or lattice structure along or within at least a portion of the body 110.

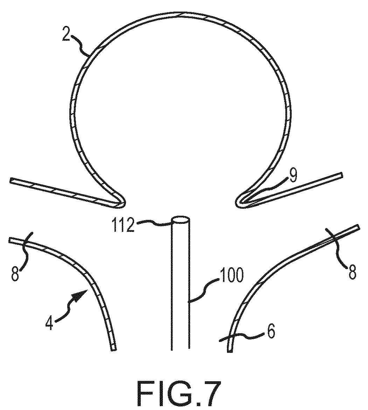

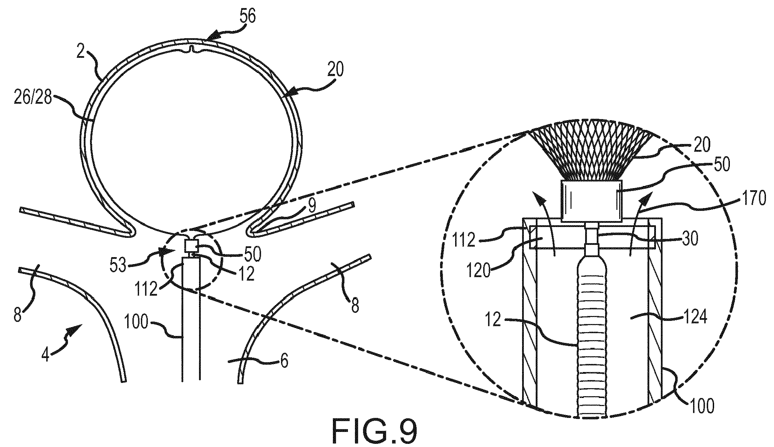

Referring now to FIGS. 7-10, with continued reference to FIGS. 1A-6D, illustrated are various stages of an exemplary method, according to one or more embodiments of the subject technology. More particularly, FIG. 7 illustrates a delivery catheter 100 near an aneurysm 2, FIG. 8 illustrates an implant 20 inserted within the aneurysm 2, FIG. 9 illustrates a stage of detachment in progress, and FIG. 10 illustrates a stage following detachment of the implant 20 from the pusher wire 12.

According to some embodiments, as shown in FIG. 7, the delivery catheter 100 is advanced to place its distal end 112 in the vicinity of a target implant site (e.g., an aneurysm 2). In addition to the components and steps shown herein, other components and stages may also be employed. For example, the delivery catheter 100 may be guided to the target implant site by a guide wire and/or a guide catheter, according to known techniques.

According to some embodiments, as shown in FIG. 8, the implant 20 can be delivered to the target implant site. For example, as shown in FIG. 8, the implant 20 can be inserted within the aneurysm to a fully deployed state. As further shown in FIG. 8, a stage in which the implant 20 is brought to the target implant site can correspond to an axial alignment of the pusher wire and the delivery catheter 100. In particular, the detachment zone 30 can be aligned with the electrode 120 when the implant is advanced out of the delivery catheter 100 and placed at the target implant site. Alternatively or in combination, the implant 20 may be placed at the target implant site, and the delivery catheter 100 may be subsequently advanced or retracted relative to the pusher wire 12 to properly align the electrode 120 with the detachment zone 30 while the pusher wire 12 holds the implant 20 steady. Alignment of the electrode 120 with the detachment zone 30 may be facilitated by components providing visualization. For example, a radiopaque marker of the delivery wire 100 can be aligned with a radiopaque marker of the pusher wire 12 and/or the implant 20 while in a configuration that corresponds to proper alignment (e.g., axial alignment) of the electrode 120 with the detachment zone 30.

According to some embodiments, as shown in FIG. 9, electrolytic detachment of the implant 20 from the pusher wire 12 can be achieved. One or both of the detachment zone 30 and the electrode 120 can be energized to apply electrical energy. For example, the detachment zone 30 and the electrode 120 can be energized with electrical energy of opposite polarity and pass electrical current through the radial gap between the detachment zone 30 and the electrode 120. This can be accomplished by activating a current source (e.g. the power source 46) connected to the detachment zone 30 by the delivery wire 31 and/or activating a current source connected to the electrode 120 by the connecting wire 126. By further example, while one of the detachment zone 30 and the electrode 120 are energized, the other is energized with an opposite polarity or grounded. According to some embodiments, during operation, the detachment zone 30 and the electrode 120 can each be multifunctional. For example, each can serve as either an active electrode or a ground electrode at different points in time as the treatment proceeds. By further example, each can serve as either a cathode or an anode at different points in time as the treatment proceeds. If desired, during the period of time that a voltage differential is formed, the polarity can be switched once or repeatedly, to create currents traveling in either direction across the gap between the detachment zone 30 and the electrodes 120.

According to some embodiments, as shown in FIG. 9, fluid flow 170 can be provided during electrolytic detachment of the implant 20 from the pusher wire 12. For example, an infusion of fluid from the fluid source 150 by the pump 160 can be provided via the delivery catheter 100 to the gap between the detachment zone 30 and the electrode 120. The fluid flow 170 can be directed distally from the lumen 124 to a region distal to the distal end 112 of the delivery catheter 100. Alternatively the fluid flow 170 can be directed proximally into the lumen 124 from a region distal to the distal end 112 of the delivery catheter 100.

According to some embodiments, the fluid flow 170 may evacuate any bubbles that form within the gap. The formation of bubbles can also change the dielectric characteristics of the gap. For example, bubbles can serve as a dielectric material and electrically insulate the detachment zone 30 from the electrode 120. Such a condition can create a dielectric region with an undesirably high breakdown voltage. The fluid flow 170 can refresh the fluid composition within the gap to maintain a clear conduction path.

According to some embodiments, the fluid flow 170 may evacuate debris from the gap between the detachment zone 30 and the electrode 120. For example, as portions of the detachment zone 30 are released into the gap, the debris can form or facilitate a short circuit from the detachment zone 30 to the electrode 120, thereby creating a bridge across the gap and reducing the rate of electrolytic detachment of the detachment zone 30. The fluid flow 170 can remove the debris to maintain a clear pathway for electrical current between the detachment zone 30 and the electrode 120.

According to some embodiments, the fluid flow 170 can be provided during part or all of an electrolytic detachment operation. For example, the fluid flow 170 may commence before, during, or after initial application of a voltage differential between the detachment zone 30 and the delivery catheter 100. By further example, the fluid flow 170 may cease before, during, or after termination of the voltage differential.

According to some embodiments, the fluid flow 170 can be provided intermittently based on conditions existing during the electrolytic detachment process. For example, the fluid flow 170 can be provided when and/or only when the power source 46 outputs a voltage and/or current above and/or below a threshold. For example, if a controller of the power source 46 detects an increase (e.g., short circuit) or decrease (e.g. open circuit) of current flow between the detachment zone 30 and the electrode 120, the fluid flow 170 can be controllably provided until the current flow normalizes to a desired value or range of values, representative of efficient electrolytic corrosion. The flow of fluid can be continuous throughout a stage or an entirety of a process. The flow can have an increased rate during portions of a process to remove debris and reduce thrombus formation.

According to some embodiments, as shown in FIG. 10, full corrosion of the detachment zone 30 results in the implant 20 being entirely separated from the pusher wire 12. Upon detachment, the fluid flow 170 can cease, and the pusher wire 12 and a delivery catheter 10 can be attracted away from the target implant site and out of the patient.

Embodiments disclosed herein can be used in veterinary or human medicine and more particularly, for the endovascular treatment of intracranial aneurysms and acquired or innate arteriovenous blood vessel deformities and/or fistulas and/or for the embolization of tumors.

The apparatus and methods discussed herein are not limited to the deployment and use of an occluding device within any particular vessels, but can include any number of different types of vessels. For example, in some aspects, vessels can include arteries or veins. In some aspects, the vessels can be suprathoracic vessels (e.g., vessels in the neck or above), intrathoracic vessels (e.g., vessels in the thorax), subthoracic vessels (e.g., vessels in the abdominal area or below), lateral thoracic vessels (e.g., vessels to the sides of the thorax such as vessels in the shoulder area and beyond), or other types of vessels and/or branches thereof.

In some aspects, the stent delivery systems disclosed herein can be deployed within superthoracic vessels. The suprathoracic vessels can include at least one of intracranial vessels, cerebral arteries, and/or any branches thereof. In some aspects, the stent delivery systems disclosed herein can be deployed within intrathoracic vessels. The intrathoracic vessels can include the aorta or branches thereof. In some aspects, the stent delivery systems disclosed herein can be deployed within subthoracic vessels. In some aspects, the stent delivery systems disclosed herein can be deployed within lateral thoracic vessels.

The foregoing description is provided to enable a person skilled in the art to practice the various configurations described herein. While the subject technology has been particularly described with reference to the various figures and configurations, it should be understood that these are for illustration purposes only and should not be taken as limiting the scope of the subject technology.

There may be many other ways to implement the subject technology. Various functions and elements described herein may be partitioned differently from those shown without departing from the scope of the subject technology. Various modifications to these configurations will be readily apparent to those skilled in the art, and generic principles defined herein may be applied to other configurations. Thus, many changes and modifications may be made to the subject technology, by one having ordinary skill in the art, without departing from the scope of the subject technology.

A phrase such as "an aspect" does not imply that such aspect is essential to the subject technology or that such aspect applies to all configurations of the subject technology. A disclosure relating to an aspect may apply to all configurations, or one or more configurations. An aspect may provide one or more examples of the disclosure. A phrase such as "an aspect" may refer to one or more aspects and vice versa. A phrase such as "an embodiment" does not imply that such embodiment is essential to the subject technology or that such embodiment applies to all configurations of the subject technology. A disclosure relating to an embodiment may apply to all embodiments, or one or more embodiments. An embodiment may provide one or more examples of the disclosure. A phrase such "an embodiment" may refer to one or more embodiments and vice versa. A phrase such as "a configuration" does not imply that such configuration is essential to the subject technology or that such configuration applies to all configurations of the subject technology. A disclosure relating to a configuration may apply to all configurations, or one or more configurations. A configuration may provide one or more examples of the disclosure. A phrase such as "a configuration" may refer to one or more configurations and vice versa.

It is understood that the specific order or hierarchy of steps in the processes disclosed is an illustration of exemplary approaches. Based upon design preferences, it is understood that the specific order or hierarchy of steps in the processes may be rearranged. Some of the steps may be performed simultaneously. The accompanying method claims present elements of the various steps in a sample order, and are not meant to be limited to the specific order or hierarchy presented.

As used herein, the phrase "at least one of" preceding a series of items, with the term "and" or "or" to separate any of the items, modifies the list as a whole, rather than each member of the list (i.e., each item). The phrase "at least one of" does not require selection of at least one of each item listed; rather, the phrase allows a meaning that includes at least one of any one of the items, and/or at least one of any combination of the items, and/or at least one of each of the items. By way of example, the phrases "at least one of A, B, and C" or "at least one of A, B, or C" each refer to only A, only B, or only C; any combination of A, B, and C; and/or at least one of each of A, B, and C.

Terms such as "top," "bottom," "front," "rear" and the like as used in this disclosure should be understood as referring to an arbitrary frame of reference, rather than to the ordinary gravitational frame of reference. Thus, a top surface, a bottom surface, a front surface, and a rear surface may extend upwardly, downwardly, diagonally, or horizontally in a gravitational frame of reference.

Furthermore, to the extent that the term "include," "have," or the like is used in the description or the claims, such term is intended to be inclusive in a manner similar to the term "comprise" as "comprise" is interpreted when employed as a transitional word in a claim.

The word "exemplary" is used herein to mean "serving as an example, instance, or illustration." Any embodiment described herein as "exemplary" is not necessarily to be construed as preferred or advantageous over other embodiments.

A reference to an element in the singular is not intended to mean "one and only one" unless specifically stated, but rather "one or more." Pronouns in the masculine (e.g., his) include the feminine and neuter gender (e.g., her and its) and vice versa. The term "some" refers to one or more. Underlined and/or italicized headings and subheadings are used for convenience only, do not limit the subject technology, and are not referred to in connection with the interpretation of the description of the subject technology. All structural and functional equivalents to the elements of the various configurations described throughout this disclosure that are known or later come to be known to those of ordinary skill in the art are expressly incorporated herein by reference and intended to be encompassed by the subject technology. Moreover, nothing disclosed herein is intended to be dedicated to the public regardless of whether such disclosure is explicitly recited in the above description.

While certain aspects and embodiments of the subject technology have been described, these have been presented by way of example only, and are not intended to limit the scope of the subject technology. Indeed, the novel methods and systems described herein may be embodied in a variety of other forms without departing from the spirit thereof. The accompanying claims and their equivalents are intended to cover such forms or modifications as would fall within the scope and spirit of the subject technology.

## Claims

1. A system for delivering an implant, comprising:
a catheter having a lumen;
a core member extending through the lumen;
an implant attached to the core member by an electrolytic detachment junction, the detachment junction being radially adjacent to an electrode within the lumen; and
a pump in fluid communication with a distal end region of the catheter through the lumen, the pump being configured to provide a flow of a fluid between the return electrode and the detachment junction.

2. The system of claim 1, wherein the return electrode is attached to an inner surface of the catheter.

3. The system of claim 1, wherein the electrode forms a ring annularly extending along an inner surface of the catheter.

4. The system of claim 1, wherein the core member is disposed along a central axis of the catheter.

5. The system of claim 1, further comprising a stabilization member extending radially into the lumen from an inner surface of the catheter and contacting the core member.

6. The system of claim 1, wherein the return electrode and the detachment junction are electrically connected to a power source.

7. The system of claim 1, wherein the detachment junction is electrolytically corrodible.

8. The system of claim 1, wherein the detachment junction is of a material that is more susceptible to electrolytic corrosion than a material of the core member or a material of the implant.

9. The system of claim 1, wherein the return electrode is disposed on a distal end of a conductive return path member extending along the catheter.

10. The system of claim 1, wherein at least a portion of the core member is electrically insulated on an outer surface thereof.

11. The system of claim 1, wherein a proximal portion of the core member, proximal to the detachment junction, has a cross-sectional dimension greater than a cross-sectional dimension of the detachment junction.

12. A method of detaching an implant, comprising:
positioning the implant, the implant being attached to a core member having a detachment junction;
positioning a catheter such that a return electrode of the catheter is radially adjacent to the detachment junction; and
applying a voltage potential across the electrolytic detachment junction and the return electrode and, while applying the voltage potential, flushing a fluid between the detachment junction and the return electrode through the lumen of the catheter.

13. The method of claim 12, wherein applying the voltage potential comprises:
applying a first charge to the detachment junction via the core member, and
applying a second charge, opposite the first charge, to the return electrode.

14. The method of claim 12, wherein positioning the return electrode comprises positioning the return electrode radially about the detachment junction.

15. The method of claim 12, wherein positioning the implant comprises advancing the implant through a lumen of the catheter.

16. The method of claim 12, wherein positioning the return electrode comprises advancing the catheter along the core member.

17. The method of claim 12, wherein applying the voltage potential comprises applying the voltage potential until the detachment junction has corroded.

18. The method of claim 12, wherein the voltage is applied until the implant is separated from the core member.

19. The method of claim 12, wherein the fluid is flushed until the implant is detached from the core member.
